# EUROPEAN PATENT APPLICATION

(11) **EP 3 113 115 A1**
(43) Date of publication of application: **04.01.2017**
(21) Application number: 16177710.7
(22) Date of filing: 04.07.2016
(51) Int. Cl.: G06T 17/20, A61B 5/00, A61B 5/01, G06T 7/00

(54) **THERMOGRAPHIC IMAGING SYSTEM**

(30) Priority: 03.07.2015 GB 201511719
(71) Applicant: Williams, Justin, London W14 9DJ (GB); Fletcher, Simon, Eversham, Worcestershire WR11 2LY (GB)
(72) Inventor: Williams, Justin, London W14 9DJ (GB); Fletcher, Simon, Eversham, Worcestershire WR11 2LY (GB)
(74) Representative: Wynne-Jones, Laine and James LLP

(57) **Abstract**

A thermographic imaging apparatus, comprising a thermographic image input and an analysis module for analysing one or more thermographic images of an area of interest according to predetermined criteria, the apparatus including an image processing module (14) configured to generate a two-dimensional image mesh (16) comprised of a plurality of segments defined by a plurality of interconnected image points, and superimpose said mesh (16) on a displayed thermographic image (10), said mesh (16) being adjustable by a user by selectively moving one or more of said image points relative to said image (10), wherein segments of said mesh can be individually selected by a user so as to select a corresponding portion of said area of interest and data representative of the location within said area of interest of said selected portion communicated to said analysis module, said analysis module being configured to change said predetermined criteria in respect of said selected portion to be used in said analysis.

## Description

### FIELD OF THE INVENTION

This invention relates generally to a thermographic imaging system and, more particularly but not necessarily exclusively, to an apparatus and method for imaging and analysing selected areas of a thermographic image.

### BACKGROUND

Thermographic imaging is used in many different fields. For example, maintenance technicians use thermography to locate overheating joints, and power lines, which are a sign of impending failure. Building construction technicians use thermography to identify heat leaks in faulty thermal insulation and can use the results to improve the efficiency of heating and air-conditioning units. Furthermore, some physiological changes in human beings and other warm-blooded animals can be monitored with thermal imaging during clinical assessment and diagnostics. For example, thermography can be used for breast screening, allergy detection, and in veterinary use.

Thermographic cameras detect radiation in the infrared range of the electromagnetic spectrum (roughly 9 - 14µm) and produce images of that radiation. The amount of radiation emitted by an object increases with temperature; therefore, thermography allows one to see variations in temperature and, when viewed through a thermal imaging camera, warm objects or areas stand out well against cooler backgrounds.

Thermographic image processing systems can be used to receive a thermographic image of a predetermined area of interest and identify portions or objects therein having a temperature above a predetermined threshold, for example. That threshold is set within the analysis software and applied indiscriminately to the whole area of interest, or to pre-programmed sections of the image associated with a threshold value or other predetermined analysis parameter.

However, a problem arises if accurate analysis of the area of interest necessitates the changing of a parameter within the analysis software in respect of just a small portion of the area of interest. For example, consider the case where a thermographic image processing system is being used to perform thermal analysis in respect of an area of interest which includes a number of pre-programmed sections, and each section is assigned some predetermined temperature threshold, or other thermal analysis parameter, against which the required thermal analysis of a respective section is performed. This process works well if the analysis parameters for all sections of an area of interest remains the same. However, there are circumstances in which it might be known, or suspected, that a particular problem, or other definitive factor, exists within a small portion of one section, in which case it may be highly desirable to be able to dynamically change the analysis parameters for a small, selected portion of a particular section of the area of interest without changing the analysis parameters for the remainder of that section, and also to be able to perform this process, using the same thermographic image processing system, in respect of multiple different areas of interest, each of which may have different portions of different sections which are known or suspected to have a particular issue associated therewith. Current thermographic imaging systems do not effectively facilitate this level of flexibility and utility.

Thus, it is an object of aspects of embodiments of the present invention to address at least some of these issues.

### STATEMENTS OF INVENTION

In accordance with an aspect of the present invention, there is provided a thermographic imaging apparatus, comprising a thermographic image input and an analysis module for analysing one or more thermographic images of an area of interest according to predetermined criteria, the apparatus including an image processing module configured to generate a two-dimensional image mesh comprised of a plurality of segments defined by a plurality of interconnected image point, and superimpose said mesh on a displayed thermographic image, said mesh being adjustable by a user by selectively moving one or more of said image points relative to said image so as to define said area of interest by the area of the mesh on said image, wherein segments of said mesh can be individually selected by a user so as to select a corresponding portion of said area of interest and data representative of the location within said area of interest said selected portion communicated to said analysis module, said analysis module being configured to change said predetermined criteria in respect of said selected portion to be used in said analysis.

In one exemplary embodiment, the mesh may include at least one segment enclosed therein which is independently adjustable relative to the other of said segments, optionally by means of a plurality of image points defining said segment, each of said image points being moveable.

The predetermined criteria may comprise a plurality of predetermined threshold values, each of which is associated with a respective mesh segment. The thermal analysis module may be configured to reduce the respective predetermined threshold value associated with each selected mesh segment.

The analysis module may be configured to determine a temperature difference between each area of a first thermographic image associated with a respective mesh segment with the corresponding area of a second thermographic image of the same area of interest captured from substantially the same viewpoint and at a different time, and generate a value representative of said temperature difference for each segment. The predetermined criteria may comprise temperature difference values, each assigned to a respective segment, and said thermal analysis module is configured to compare, for each segment, the generated temperature difference with the respective predetermined temperature difference value, and provide an indication of a result of said comparison. The respective predetermined temperature difference value may, in one exemplary embodiment, be reduced for each selected mesh segment.

Another aspect of the invention extends to a method of processing one or more thermographic images of an area of interest according to predetermined criteria, the method comprising generating a two-dimensional image mesh comprised of a plurality of segments defined by a plurality of interconnected image point, superimposing said mesh on a displayed thermographic image, said mesh being adjustable by a user by selectively moving one or more of said image points relative to said image so as to define said area of interest by the area of the mesh on said image, selecting one or more segments of said mesh so as to select a corresponding portion of said area of interest, communicating data representative of the location within said area of interest communicated to said analysis module, changing said predetermined criteria in respect of said selected portion, and analysing data representative of each portion of said area of interest defined by a respective mesh segment against the predetermined criteria assigned thereto to generate an output.

Yet another aspect of the invention extends to an image processing and analysis module for apparatus according to any of claims 1 to 8, said module being configured to generate a two-dimensional image mesh comprised of a plurality of segments defined by a plurality of interconnected image point, superimpose said mesh on a displayed thermographic image, said mesh being adjustable by a user by selectively moving one or more of said image points relative to said image so as to define said area of interest by the area of the mesh on said image, wherein segments of said mesh can be individually selected by a user so as to select a corresponding portion of said area of interest and data representative of the location within said area of interest of said selected portion communicated to said analysis module, and change said predetermined criteria in respect of said selected portion to be used in said analysis.

These and other aspects of the invention will become apparent from the following detailed description.

### DETAILED DESCRIPTION

Embodiments of the present invention will now be described by way of examples only and with reference to the accompanying drawings, in which:
Figure 1 is a schematic block diagram illustrating some of the principal components of a thermographic imaging system according to an exemplary embodiment of the present invention;
Figure 2 is a diagrammatic illustration of a two-dimensional mesh that may be generated by the thermal analysis module in apparatus according to an exemplary embodiment of the present invention;
Figure 3 is a flow diagram illustrating some of the steps of a thermographic imaging method according to an exemplary embodiment of the present invention; and
Figure 4 is a flow diagram illustrating some of the additional steps of a thermographic imaging method according to an exemplary embodiment of the present invention.

In a typical thermographic analysis process, it is known to compare two or more thermographic images in order to identify aspects of specific concern that might indicate a defect or warrant further investigation. In many cases, the two or more thermographic images may be:
a) of the same area of interest, captured from the same interest, at different times: for example, before and after a predetermined cooling period; or
b) of similar areas of interest (in respect of different subjects), captured from the same viewpoint and at the same time.

It will be appreciated that, as the fields in which thermographic analysis can be used are numerous, so are the so-called "areas of interest" that may be required to be analysed. For example, the area of interest may comprise a component or group of components in an industrial machine, in which persistent "hotspots" may indicate a problem or imminent mechanical failure. However, for the purposes of the following description, an exemplary embodiment in relation to human breast screening will be described, although it will be understood by a person skilled in the art that the principles described herein are equally applicable to thermographic analysis in other, non-medical fields, and the present invention, in its broadest context, is not necessarily intended to be limited in this regard.

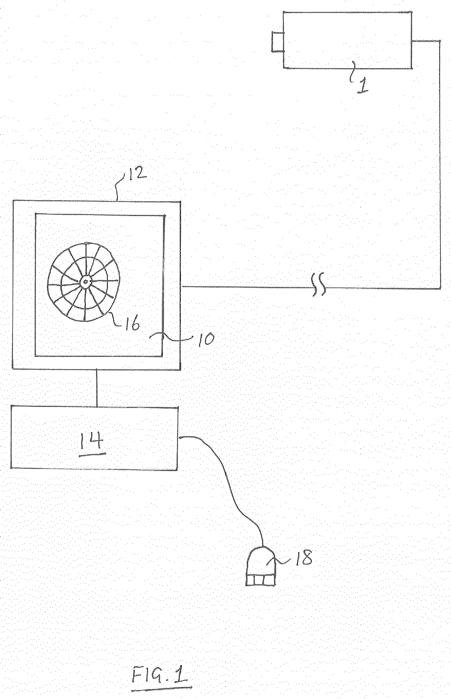

Thus, for the purposes of describing one exemplary embodiment of the present invention, a thermographic camera (reference numeral 1 - Figure 1) is used to capture a first thermographic image of a subject's thorax or chest, including the breasts, from a specified viewpoint, and a second thermographic image of the same subject's thorax from the same viewpoint after a predetermined cooling period, e.g. 10 minutes.

It will be well known to a person skilled in the art that thermographic images depict the captured image in terms of colours representing temperatures or temperature ranges of each element of the subject. In general, the colours range from red, showing the highest temperatures, to dark blue showing the lowest temperatures, through a spectrum progressively depicting intermediate temperatures between the highest and lowest. Thus, the resultant "image", rather than depicting a visual or optical representation of the subject area, actually represents the subject area in terms of its thermal (rather than visual) properties. The resultant image may also be converted into grey scale for some applications, although the distinction is not of fundamental importance for the purposes of this description. Thus, references herein to thermographic images is intended to cover colour and/or grey scale images captured using a thermographic camera, and the present invention is not intended to be any further limited in this regard.

Referring now to Figure 1 of the drawings, a first 10 of the captured images is displayed to a user on a computer monitor 12, and an image processing module 14 generates a two-dimensional image mesh 16 and superimposes the mesh onto the image 10. The mesh 16 is intended to be a skeletal general representation of the area of interest, in terms of its shape, and configured to allow a user to adjust its profile so as to exactly fit the shape of the subject area of interest.

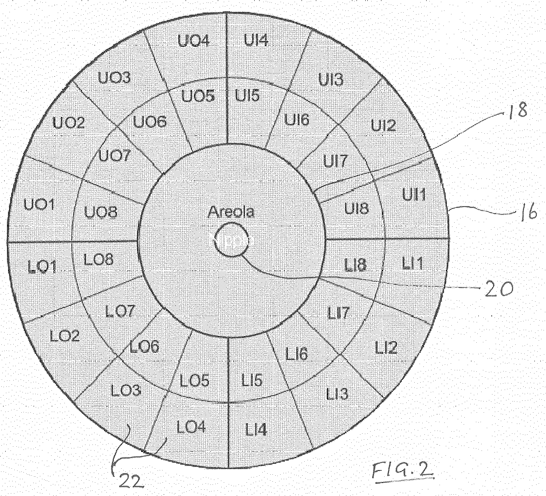

Thus, as shown in Figure 2 of the drawings, the mesh 16 for this particular exemplary embodiment of the invention is generally circular in shape and comprises an outer circle and three concentric inner circles, of progressively smaller diameter. Overall, it can be seen that the mesh is of a configuration that has the general appearance of a female breast, with the two inner circles 18, 20 being representative of the areola and nipple respectively. The portion of the mesh between the "areola" and its periphery is divided into 32 segments 22 by equi-distant radial connecting lines. Where each connecting line meets the "areola" 18 and the outer circle, an image point or "handle" is defined, which is a point on the mesh that can be selected and moved or dragged by the user in order to adjust the size and shape of the mesh to the exact size and shape of the subject breast and, crucially, also drag and shape the "areola" to ensure that it exactly matches the areola of the subject breast and that the nipple portion 20 is correctly placed within the superimposed image.

Once the mesh 16 has been correctly adjusted to match the subject area of interest, the analysis process can begin. In general, part of the analysis process in this case involves a comparison of the temperature in each segment of the subject area (defined by the positioning of the superimposed mesh) against:
a) the same segment of the same breast before and after cooling;
b) the same segment of the other breast, at the same time (either before or after cooling).

Thus, what is being measured and compared is a change or difference in temperature, referred to herein as Δ (delta) T.

Depending on the field to which the present invention is being applied, the "normal" delta T would represent the expected change or difference in temperature, and a predetermined threshold for delta T is set for each segment of the subject area that represents this value, within a predetermined tolerance range.

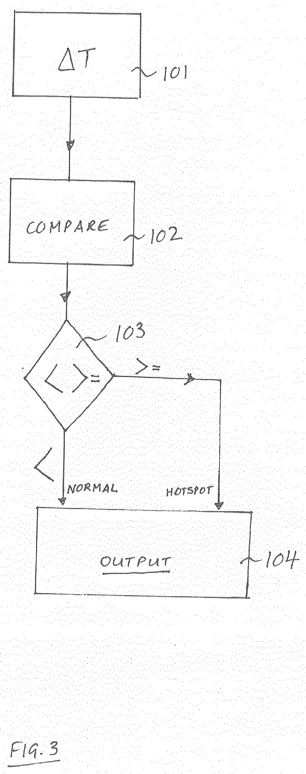

In this exemplary embodiment of the invention, delta T represents the change in temperature of each segment of the same breast before and after the cooling period. Thus, referring to Figure 3 of the drawings, under normal circumstances, the analysis module operates as follows:
1. Determine delta T for each segment of each mesh (left and right) (step 101)
2. Compare each delta T with a predetermined threshold value, e.g. 2°C (step 102)
3. Identify segments of the breast for which delta T is less than the predetermined threshold value ("hotspots"), i.e. those segments of the breast that have not cooled by at least 2°C during the cooling period (step 103); and
4. Provide a visual display of the location and status of at least any identified "hotspots" (step 104).

The thermographic images have the ability to depict vascular patterns, i.e. patterns of blood vessels within the breast. These can be clearly seen within the images themselves. Thus, it is possible, assessing the images, to identify the location of abnormal vascular patters. Abnormal vascular patterns include the following indicators:
- Bulge or edge sign
- Disturbed asymmetrical vascular pattern
- Vascular fragmentation
- Inferior vascularity
- Loop vascular pattern
- Transverse vascular pattern
- Spike/inverted "V" vascular pattern
- Vascular spider/star vascular pattern
- Inflammatory vascular pattern
- Vascular chaos

Some of these indicators may have characteristics that are immediately identifiable, others are identifiable by differences between the vascular patterns in the right and left breast (which are expected to be substantially "symmetrical"). They may be identifiable by image recognition techniques within the analysis module, but in its simplest form, they may simply be identified by a user's visual assessment of the thermographic images provided at the start of the process.

Either way, if an abnormal vascular pattern is identified, it is desirable to be able to change the threshold of delta T for the segments affected, so as to ensure that any "hotspots" overlapping an abnormal vascular pattern are reliably identified. Depending on the abnormality identified, one or more individual segments or quadrants may be affected, or half or all of the breast may be affected. Exemplary embodiments of the invention, enable the affected segments within the mesh to be selected on the screen as required, to identify the affected areas of the breast. In exemplary embodiments of the present invention, where the vascular analysis is performed automatically, such selection may also be performed automatically based on the analysis results. However, once again, in its simplest form, where a user has identified the location of any abnormal vascular patterns, the system may be configured to enable the user to select the affected areas by selecting the respective areas of the mesh on the screen by pointing and clicking with their computer mouse or other input device (e.g. stylus or finger for touch screen monitors).

Once selected segments of the subject area have been selected, data representative thereof is used by the analysis module to change the delta T threshold for just those segments before running the thermal analysis. In one exemplary embodiment, for example, the threshold may be reduced from 2°C to 1°C. However, these values are very specific to the field in which the invention is being used, and the present invention is not necessarily intended to be limited in this regard.

In addition, a subject may have identified a potential issue in a particular location of a breast, for example a lump or discomfort, and if this is the case, the user may wish to reduce the delta T threshold for that area. Once again, the associated segments of the mesh can be selected so as to change delta T for the thermal analysis in relation to the areas associated with the selected segments.

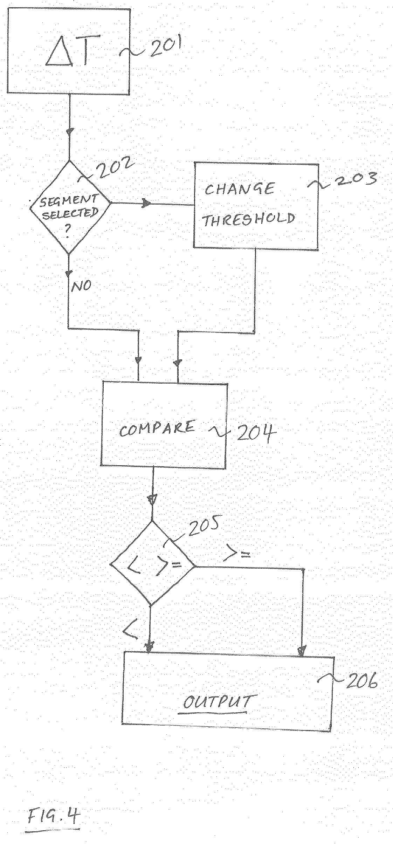

Thus, referring to Figure 4 of the drawings, a more complex analysis method according to an exemplary embodiment of the invention comprises the following steps:
1. Determine delta T for each segment of each mesh (left and right) (step 201)
2. Determine which, if any, segments of the mesh are selected (step 202).
3. Change the predetermined threshold value for just the selected mesh segments (step 203).
4. Compare delta T for each mesh segment with its predetermined threshold value (step 204).
5. Identify segments of the breast for which delta T is less than the predetermined threshold value ("hotspots"), i.e. those segments of the breast that have not cooled by at least the set threshold value during the cooling period (step 205); and
6. Provide a visual display of the location and status of at least any identified "hotspots" (step 206).

In one exemplary embodiment, the threshold value for each selected segment is changed from a default value to another predetermined threshold value. However, in alternative exemplary embodiments, when one or more segments of the mesh is selected, the user may be given two or more options as to what the threshold value should be changed to, and may even be given the opportunity to enter their own threshold value in some embodiments.

Embodiments of the present invention have a number of advantages, compared with the prior art, which include:
- The ability to dynamically change the threshold value(s) within the thermal analysis module by selecting potential areas of concern by selecting one or more segments of an adjustable mesh which is superimposed on a thermographic image of the subject area.
- The provision of an adjustable mesh, which has areas within it that can be independently adjusted. In the specific exemplary embodiment described above, the area of the mesh representative of the areola can be independently adjusted within the body of the mesh to exactly match that of the thermographic image on which it is superimposed, thereby significantly increasing the accuracy of the subsequent thermal analysis.

It will be apparent from the foregoing description that modifications and variations can be made to the described embodiments without departing from the scope of the invention as claimed.

## Claims

1. A thermographic imaging apparatus, comprising a thermographic image input and an analysis module for analysing one or more thermographic images of an area of interest according to predetermined criteria, the apparatus including an image processing module configured to generate a two-dimensional image mesh comprised of a plurality of segments defined by a plurality of interconnected image point, and superimpose said mesh on a displayed thermographic image, said mesh being adjustable by a user by selectively moving one or more of said image points relative to said image so as to define said area of interest by the area of the mesh on said image, wherein segments of said mesh can be individually selected by a user so as to select a corresponding portion of said area of interest and data representative of the location within said area of interest of said selected portion communicated to said analysis module, said analysis module being configured to change said predetermined criteria in respect of said selected portion to be used in said analysis.

2. Apparatus according to claim 1, wherein said mesh includes at least one segment enclosed therein which is independently adjustable relative to the other of said segments.

3. Apparatus according to claim 2, wherein said mesh includes at least one segment enclosed therein which is independently adjustable relative to the other of said segments by means of a plurality of image points defining said segment, each of said image points being moveable.

4. Apparatus according to any of the preceding claims, wherein said predetermined criteria comprise a plurality of predetermined threshold values, each of which is associated with a respective mesh segment.

5. Apparatus according to claim 4, wherein said thermal analysis module is configured to reduce the respective predetermined threshold value associated with each selected mesh segment.

6. Apparatus according to any of the preceding claims, wherein the analysis module is configured to determine a temperature difference between each area of a first thermographic image associated with a respective mesh segment with the corresponding area of a second thermographic image of the same area of interest captured from substantially the same viewpoint and at a different time, and generate a value representative of said temperature difference for each segment.

7. Apparatus according to claim 6, wherein said predetermined criteria comprise temperature difference values, each assigned to a respective segment, and said thermal analysis module is configured to compare, for each segment, the generated temperature difference with the respective predetermined temperature difference value, and provide an indication of a result of said comparison.

8. Apparatus according to claim 6 or claim 7, wherein the respective predetermined temperature difference value is reduced for each selected mesh segment.

9. A method of processing one or more thermographic images of an area of interest according to predetermined criteria, the method comprising generating a two-dimensional image mesh comprised of a plurality of segments defined by a plurality of interconnected image point, superimposing said mesh on a displayed thermographic image, said mesh being adjustable by a user by selectively moving one or more of said image points relative to said image so as to define said area of interest by the area of the mesh on said image, selecting one or more segments of said mesh so as to select a corresponding portion of said area of interest, communicating data representative of the location within said area of interest communicated to said analysis module, changing said predetermined criteria in respect of said selected portion, and analysing data representative of each portion of said area of interest defined by a respective mesh segment against the predetermined criteria assigned thereto to generate an output.

10. A method according to claim 9, wherein said mesh includes at least one segment enclosed therein which is independently adjustable relative to the other of said segments, and optionally wherein said mesh includes at least one segment enclosed therein which is independently adjustable relative to the other of said segments by means of a plurality of image points defining said segment, each of said image points being moveable.

11. A method according to claim 9 or claim 10, wherein said predetermined criteria comprise a plurality of predetermined threshold values, each of which is associated with a respective mesh segment, and optionally comprising the step of reducing the respective predetermined threshold value associated with each selected mesh segment.

12. A method according to any of claims 9 to 11, comprising the steps of determining a temperature difference between each area of a first thermographic image associated with a respective mesh segment with the corresponding area of a second thermographic image of the same area of interest captured from substantially the same viewpoint and at a different time, and generating a value representative of said temperature difference for each segment.

13. A method according to claim 12, wherein said predetermined criteria comprise temperature difference values, each assigned to a respective segment, and the method comprises the steps of comparing, for each segment, the generated temperature difference with the respective predetermined temperature difference value, and providing an indication of a result of said comparison.

14. A method according to claim 12 or claim 13, wherein the respective predetermined temperature difference value is reduced for each selected mesh segment.

15. An image processing and analysis module for apparatus according to any of claims 1 to 8, said module being configured to generate a two-dimensional image mesh comprised of a plurality of segments defined by a plurality of interconnected image point, superimpose said mesh on a displayed thermographic image, said mesh being adjustable by a user by selectively moving one or more of said image points relative to said image so as to define said area of interest by the area of the mesh on said image, wherein segments of said mesh can be individually selected by a user so as to select a corresponding portion of said area of interest and data representative of the location within said area of interest of said selected portion communicated to said analysis module, and change said predetermined criteria in respect of said selected portion to be used in said analysis.
